# EUROPÄISCHE PATENTSCHRIFT

(11) **EP 3 102 309 B1**
(45) Veröffentlichungstag und Bekanntmachung des Hinweises auf die Patenterteilung: **05.12.2018**
(21) Anmeldenummer: 15704458.7
(22) Anmeldetag: 29.01.2015
(51) Int. Cl.: B01D 53/04, C07C 41/01, C07C 41/36, C07C 29/74, C10K 1/00, C10G 2/00

(54) **VERFAHREN ZUR HERSTELLUNG HÖHERMOLEKULARER VERBINDUNGEN AUS SYNTHESEGAS UNTER VERWENDUNG EINES INDIREKT BEHEIZTEN CO2-TSA**
METHOD FOR PRODUCING A HIGH-MOLECULAR COMPOUND FROM SYNTHESIS GAS USING AN INDIRECTLY HEATED CO2 TSA
MÉTHODE POUR PRODUIRE DES COMPOSES À POIDS MOLÉCULAIRE ÉLEVÉS EN UTILISANT UN TSA-CO2 INDIRECTEMENT CHAUFFÉ

(30) Priorität: 04.02.2014 EP 14000399
(43) Veröffentlichungstag der Anmeldung: 14.12.2016
(73) Patentinhaber: Linde Aktiengesellschaft, 80331 München (DE)
(72) Erfinder: VOSS, Christian, 82538 Geretsried (DE); PESCHEL, Andreas, 82515 Wolfratshausen (DE); SCHÜRER, Benedikt, 82049 Pullach (DE); SALAZAR DUARTE, Gabriel, 80336 München (DE)
(86) Internationale Anmeldenummer: PCT/EP2015/000172
(87) Internationale Veröffentlichungsnummer: WO 2015/117738

(56) Entgegenhaltungen:
- WO-A1-2008/143966
- WO-A1-2013/062800
- WO-A2-02/26676
- WO-A2-2009/075942
- US-A1- 2013 030 063

## Beschreibung

Die Erfindung betrifft ein Verfahren zur Herstellung eines oder mehrerer Reaktionsprodukte mittels einer aufbauenden Reaktion, bei der aus den niedermolekularen Verbindungen eines Synthesegases zumindest teilweise höhermolekulare Verbindungen gebildet werden.

Als niedermolekulare Verbindung wird in verschiedenen Bereichen eine Klasse von Stoffen mit niedriger Molekülmasse bezeichnet. In der Regel bilden sie die Gegengruppe zu größeren, höhermolekularen Verbindungen, die höhere Molekülmasse als die niedermolekulare Verbindung haben. Unter den niedermolekularen Verbindungen eines Synthesegases sind hier vorzugsweise CO und H2 gemeint, aus denen mehrere verschiedene höhermolekulare Verbindungen gebildet werden können, wie z.B Methanol, Ethen usw. Es kann auch sein, dass ein oder mehrere aus CO und H2 gebildete erste Produkte in ein oder mehrere weitere Produkte, die im Vergleich zu den ersten Produkten noch höhere höhermolekulare Verbindungen haben, umgesetzt werden. Ein Beispiel hierfür ist die Hydroformylierung von Alkenen zur Erzeugung von Aldehyden/Ketonen (Alken + H₂+CO -> Aldehyd, H₂:CO = 1, kein CO₂) sowie die Hydroformylierung von Alkenen zur Erzeugung von Alkoholen (Alken + 2H₂+CO -> Alkohol, H₂:CO = 2, kein CO₂).

Als weiteres Beispiel kann Dimethylether (DME) durch eine Direktsynthese mittels Dehydratisierung von Methanol hergestellt werden. Hierfür wird Methanol aus einem Synthesegas mit einer stöchiometrischen Zahl SN= (x_{H2}-x_{CO2})/(x_{CO}+x_{CO2}) von ungefähr 2 erzeugt. Dieser zweistufige Prozess ist z.B. in "Dimethyl Ether Technology and Markets. CHEMYSTEMS PERP Program, Nexant Inc., November 2008" sowie in "Japan DME Forum. 2007. DME Handbook. Japan DME Forum, Tokyo" im Detail beschrieben.

Alternativ hierzu kann DME in einem einstufigen Prozess, der auch als Direktsynthese bezeichnet wird, aus einem Synthesegas hergestellt werden. Derartige Direktsynthesen werden z.B. im oben genannten Stand der Technik sowie in "Takashi Ogawa, Norio Inoue, Tutomu Shikada, Yotaro Ohno, 2003: Direct Dimethyl Ether Synthesis. Journal of Natural Gas Chemistry 12, 219-227" und "Lee, Seung-Ho, Cho, Wonjun, Song, Taekyong, & Ra, Young-Jin, 2009: Scale Up Study of DME Direct Synthesis Technology. In: Proceedings of the 24th World Gas Conference, Buenos Aires, Argentina" beschrieben.

Bei der Direktsynthese von DME wird ein Synthesegas enthaltend CO und H₂ in einer Einrichtung zur Synthesegaserzeugung (z.B. in einem Synthesegasreaktor wie beispielsweise ein Dampfreformer zur Dampfreformierung von Kohlenwasserstoffen, insbesondere CH₄) erzeugt und in einen DME-Reaktor eingeleitet, in dem die Direktsynthese von DME durch katalysierte Umsetzung des Synthesegases mittels eines geeigneten Katalysators durchgeführt wird, wobei ein Produktstrom oder Reaktoreffluent enthaltend DME, CO₂, H₂O, CH₃OH und nicht umgesetztes Synthesegas (CO und H₂) erzeugt wird. Das Synthesegas aus CO und H₂ kann dabei z.B. aus Erdgas hergestellt werden, beispielsweise durch Dampfreformierung: CH₄ + H₂O -> 2CO + 4H₂.

Daneben besteht die Möglichkeit, Erdgas auch durch partielle Oxidation zu Synthesegas umzusetzen: 2CH₄ + O₂ -> 2CO + 4H₂.

Ferner kann auch durch autotherme Reformierung (Kombination von Dampfreformierung und partieller Oxidation in einem Apparat) Synthesegas erzeugt werden. Die beiden Verfahren werden so miteinander kombiniert, dass der Vorteil der Oxidation (Bereitstellung von Wärmeenergie) sich mit dem Vorteil der Dampfreformierung (höhere Wasserstoffausbeute) vorteilhaft ergänzt.

Schließlich kann auch durch das sogenannte combined Reforming (Kombination von Dampfreformierung und partieller Oxidation in getrennten Apparaten) Synthesegas hergestellt werden.

Darüber hinaus kann das Synthesegas noch aus Trockenreformierung (Dry Reform) hergestellt werden oder aus verschiedenen Kombinationen der bekannten Herstellungsverfahren des Synthesegases, z.B. Reihen- oder Parallelschaltung von autothermer Reformierung oder partieller Oxidation mit Dampfreformierung oder Trockenreformierung. Das erfindungsgemäße Verfahren ist nicht auf einzelne Synthesegaserzeugungsverfahren beschränkt, das gilt auch für die Kombinationen von den geeigneten Verfahren, insofern die Erzeugung des Synthesegases erzielt wird.

Die Direktsynthese von DME erfolgt aus dem Synthesegas nach der folgenden Summengleichung: 3H₂ + 3CO -> DME + CO₂.

Gemäß der Stöchiometrie kann z.B. der Einsatz eines Synthesegases mit einem H₂/CO- Verhältnis von etwa 1:1 verwendet werden. Andere Synthesegaszusammensetzungen sind auch möglich.

Als Mechanismus wird aktuell folgender Reaktionspfad über intermediäres Methanol angenommen:

2H₂ + CO -> CH₃OH,

2CH₃OH -> DME + H₂O,

und

H₂O+CO -> CO₂+H₂ (Wassergas-Shift-Reaktion).

WO2009/075942 offenbart ein Verfahren zur Herstellung von C1 bis C4 Alkoholen aus Synthesegas und gleichzeitig auch CO2 entfernen. Der Produktstrom wird in einer Trenneinrichtung in einen kondensierte Fraktionsstrom aufweisend Alkoholen und einen Reclclestrom aufweisend CO2, CO und H2 getrennt.

WO2008143966A offenbart eine Rapid TSA, in dem der beladene Adsorbens in der Regenerationsphase durch eine indirekt thermische Welle geheizt wird.

Um die Effizienz des Kohlenstoffeinsatzes in der Direktsynthese von DME zu erhöhen und die CO₂-Emission zu verringern, ist es vorteilhaft, die Kohlenstoffkomponente CO₂, sowie insbesondere CO und CH₄ zurückzugewinnen und in die Synthesegasproduktion zurückzuführen. Die Aufgabe besteht nicht nur für Direktsynthese von DME, sondern auch allgemein für andere aufbauende Reaktionen.

Dieses Problem wird durch ein Verfahren mit den Merkmalen des Anspruchs 1 gelöst. Vorteilhafte Ausgestaltungen der Erfindung sind u.a. in den abhängigen Ansprüchen aufgezeigt. Die Merkmale der Ansprüche können in jeglicher technisch sinnvollen Art und Weise kombiniert werden, wobei hierzu auch die Erläuterungen aus der nachfolgenden Beschreibung sowie Merkmale aus den Figuren hinzugezogen werden können, die ergänzende Ausgestaltungen der Erfindung umfassen.

Gemäß Anspruch 1 ist erfindungsgemäß vorgesehen, dass ein Synthesegasstrom aufweisend CO und H₂ (siehe z.B. oben) bereitgestellt wird und in einen Reaktor eingeleitet wird, in dem eine aufbauende Reaktion durchgeführt wird. Unter dem Begriff Synthesegas wird ein Gasgemisch, insbesondere ein industriell hergestelltes Gasgemisch, verstanden, welches im Wesentlichen Kohlenstoffmonoxid und Wasserstoff und gegebenenfalls weitere Gase enthält. Aus den niedermolekularen Verbindungen des Synthesegases werden zumindest teilweise höhermolekulare Verbindungen gebildet, wobei ein Produktstrom enthaltend höhermolekulare Verbindungen, CO₂, nicht umgesetztes Synthesegas und ggf. CH₄ erzeugt wird. Hierbei werden Bestandteile des Synthesegases zumindest teilweise in Verbindungen chemisch umgesetzt, welche eine höhere Molekülmasse als die eingesetzten Bestandteile des Synthesegases haben.

Der erhaltene Produktstrom mit den höhermolekularen Verbindungen wird in einer Trenneinrichtung in einen ersten Strom aufweisend die höhermolekulare Verbindung sowie in einen zweiten Strom aufweisend CO₂ ,CO, H₂ sowie ggf. CH₄ getrennt. In einer Temperaturwechseladsorptionseinrichtung stromab der Trenneinrichtung wird mittels Temperaturwechseladsorption CO₂ aus dem zweiten Strom abgetrennt wird, wobei bei der Temperaturwechseladsorption das CO₂ an einem Adsorbens adsorbiert wird. Das mit CO₂ beladene Adsorbens wird durch Heizen des Adsorbens regeneriert, wobei CO₂ desorbiert wird.

Die Beheizung des beladenen Adsorbens erfolgt dadurch, dass zumindest teilweise indirekt Wärme eines fluiden Wärmeträgermediums auf das Adsorbens übertragen wird. Das Adsorbens kann auch dadurch beheizt werden, dass die Wärme eines fluiden Wärmeträgermediums ausschließlich indirekt auf das Adsorbens übertragen wird. Diesbezüglich ist es denkbar, dass dem Adsorber nur ein Teil der für die Regeneration benötigten Wärme durch ein Wärmeträgerfluid indirekt zugeführt und ein anderer Teil z.B. durch direkte Beheizung mit heißem Gas, wie bei dem konventionellen TSA Prozess. Das Adsorbens kann auch durch mehrere Adsorbentien gebildet sein, z.B. in Form eines Schichtbetts.

Bevorzugt wird das Adsorbens beim Adsorbieren durch indirekte Wärmeübertragung auf ein oder das fluide Wärmeträgermedium gekühlt.

Ein derartiges Temperaturwechseladsorptionsverfahren, bei dem auf einer niedrigeren Temperatur adsorbiert und bei einer höheren Temperatur, die durch die dargestellte indirekte Wärmeübertragung erzielt wird, das Adsorbens regeneriert wird, wird auch als Rapid-Temperaturwechseladsorption (RTSA für Rapid Temperature Swing Adsorption) bezeichnet. Derartige Verfahren sind z.B. aus EP1291067A2, WO2012085128A1, US8226746B2, und WO2008143966A1 bekannt.

Aufgrund der indirekten Wärmeübertragung mittels des räumlich separat geführten fluiden Wärmeträgermediums werden kürzere Zykluszeiten im Vergleich zu einer konventionellen Temperaturwechseladsorption erreicht, bei der das Adsorbens direkt durch Beaufschlagen mit heißem Regeneriergas geheizt wird.

Weiterhin erlaubt das indirekte Kühlen bei der RTSA während der Adsorption das Entfernen größerer Mengen an Verunreinigungen im Vergleich zu einer herkömmlichen TSA. Weiterhin benötigen RTSA-Anlagen regelmäßig weniger Adsorbens, was ein kompaktes Design einer solchen Anlage ermöglicht, Kosten verringert und eine Rückgewinnung der adsorbierten Komponente mit hoher Konzentration ermöglicht, da aufgrund des Verzichts auf Regeneriergas oder der Verwendung von weniger Regeneriergas keine oder eine geringere Verdünnung der desorbierten Komponenten erfolgt.

Im Vergleich zu anderen, aus dem Stand der Technik bekannten Verfahren zum Abtrennen von Komponenten eines Gasgemisches, wie z.B. die Druckwechseladsorption, Membrantrennverfahren oder Aminwäschen, erlaubt die RTSA vor allem das Abtrennen der abzutrennenden Komponente auf einem hohem Druck. Das Design eines Apparates zur Durchführung einer RTSA kann z.B. auf einem Rohrbündelwärmeübertrager basieren, wie z.B. in der EP1291067 A2 beschrieben, auf eine Mikrokanaleinrichtung (vgl. z.B. US6974496 B2), auf einem strukturierten Adsorbenskontaktor (siehe z.B. WO2008143823 A1) oder auf Hohlfasern, wie in WO2009003171 A1 beschrieben. In der Literatur beschriebene Verfahren betreffen das Entfernen von C₂₊-Fraktionen aus Erdgas (siehe WO2012118747 A1) sowie die Entfernung von CO₂ aus Rauchgas (siehe WO2012064919A1).

Der aus der Temperaturwechseladsorptionseinrichtung entnommene Strom, aus dem CO2 abgetrennt wurde, wird im Kreislauf dem Reaktor zugeführt und das Synthesegas wird vor der Einleitung in den Reaktor in die Temperaturwechseladsorptionseinrichtung geleitet. Mit dieser Ausführungsform wird die Temperaturwechseladsorptionseinrichtung nicht nur zur Entfernung von CO2 aus den zweiten Strom, sondern auch zur Entfernung von CO2 aus dem bereitgestellten Synthesegas vor der aufbauenden Reaktion genutzt, wodurch eine erhöhte Effizienz des Kohlenstoffeinsatzes sowie eine geringere CO2-Emission erzielt werden.

Bevorzugt wird das bei der Temperaturwechseladsorption adsorbierte (oder. desorbierte) CO₂ als Einsatz in einen Synthesegas-Reaktor zurückgeführt, in dem das der aufbauenden Reaktion zugrunde liegende Synthesegas erzeugt wird. Das CO₂ kann aber auch für andere Synthesen verwendet werden. Das adsorbierte CO₂ zeichnet sich dann durch eine höhere Reinheit aus als bei den meisten anderen Verfahren und weist insbesondere - bis ggf. auf Spuren - kein O₂ sowie keine Amine auf.

Weiterhin wird bevorzugt das CO₂ bei der Temperaturwechseladsorption auf einem hohem Druckniveau im Bereich von 5 bar bis 40 bar, bevorzugt 15 bar bis 25 bar zurückgewonnen, so dass im Vergleich zu anderen Reinigungsverfahren vergleichsweise weniger Kompressionsstufen verwendet werden können oder auf eine Kompression des rückzuführenden CO₂ vollständig verzichtet werden kann.

In einer bevorzugten Ausführungsform der Erfindung ist vorgesehen, dass bei einer Temperaturwechseladsorptionseinrichtung vorzugsweise lediglich CO₂ aus dem zweiten Strom abgetrennt wird und in den Synthesegas-Reaktor zurückgeführt wird, wobei der von CO₂ gereinigte zweite Strom aufweisend CO und H₂ zumindest teilweise in den Reaktor zurückgeführt wird.

Weiterhin wird gemäß einer Ausgestaltung der Erfindung bei einer Temperaturwechseladsorptionseinrichtung neben CO₂, auch CO und CH₄ in der Temperaturwechseladsorptionseinrichtung mittels Temperaturwechseladsorption aus dem zweiten Strom abgetrennt (und zwar ebenfalls auf dem oben beschriebenen hohen Druckniveau) und in den Synthesegas-Reaktor zurückgeführt, wobei der von CO₂, CO und CH₄ gereinigte H₂-reiche sowie N₂-haltige zweite Strom zumindest teilweise in den Reaktor zurückgeführt wird und/oder zumindest teilweise in einer Druckwechseladsorptionseinrichtung mittels Druckwechseladsorption weiter gereinigt wird, wobei insbesondere N₂ adsorbiert wird und insbesondere H₂ entweder zum Reaktor geführt wird, zur Entschwefelung geführt wird oder für andere Anwendungen gewonnen wird.

Weiterhin besteht die Möglichkeit einen Teilstrom des Synthesegases im Bypass an der RTSA vorbeizuführen, um so den CO₂-Gehalt des Synthesegases gezielt einzustellen.

Zusammenfassend erlaubt die erfindungsgemäße Lösung CO₂ vom Synthesegas zu trennen und in die Synthesegaserzeugung zu rezirkulieren (insbesondere bei Reformer-basierten Synthesegastechnologien) bzw. zusätzlich oder lediglich als Exportstrom zu gewinnen, wobei vergleichsweise weniger Kompressionsenergie erforderlich ist bzw. auf einen Kompressor völlig verzichtet werden kann. Weiterhin können aus dem aus der aufbauenden Reaktion zurückzuführenden Gasstrom die kohlenstoffhaltigen Komponenten auf hohem Druck zurückgewonnen werden und in die Synthesegaserzeugung zurückgeführt werden. Wasserstoff kann des Weiteren zur aufbauenden Reaktion zurückgeführt werden und/oder als Nebenprodukt gewonnen werden. Überschüssiger Dampf, der während des Prozesses erzeugt wird, kann als fluides Wärmeträgermedium für die RTSA verwendet werden oder zum Aufheizen des Wärmeträgermediums, z.B. mittels eines Wärmeübertragers. Ggf. kann ein vergleichsweise kleiner Purge- oder Spül-Strom bei der Regeneration des Adsorbens verwendet werden, um die Desorption weiter zu beschleunigen. Hierzu können methan- und/oder wasserstoffreiche Ströme verwendet werden.

Des Weiteren kann der CO₂-Gehalt des Einsatzstroms in die aufbauende Reaktion mit Vorteil gesteuert werden, ebenso wie der CO₂-Gehalt im rezirkulierten Strom. Im Reaktor erzeugtes CO₂ kann von dem Produktstrom unter hohem Druck abgetrennt werden. Zusätzlich können weitere kohlenstoffhaltige Komponenten, wie z.B. CO und CH₄ von dem Produktstrom abgetrennt werden und in die Synthesegaserzeugung unter hohem Druck zurückgeführt werden. Hierdurch kann der notwendige Purge-Strom zur Reduktion inerter Komponenten in der aufbauenden Reaktion reduziert werden. Wasserstoff kann als Nebenprodukt gewonnen werden, in Kombination mit der Rückführung der kohlenwasserstoffhaltigen Komponenten unter hohem Druck in die Synthesegaserzeugung.

Die vorliegende Erfindung kann zur Aufbereitung von Synthesegasen in weiteren Anwendungen verwendet werden, wobei der Fokus vorzugsweise auf der Reduktion des CO₂-Gehaltes des Synthesegases sowie der Rückführung des abgetrennten CO₂ in die Synthesegaserzeugung oder den Reformer liegt.

Bei der aufbauenden Reaktion werden ein oder mehrere Edukte mit niedermolekularen Verbindungen in ein oder mehrere Produkte mit höhermolekularen Verbindungen umgesetzt. Bevorzugt sind die Edukte Kohlenmonoxid und Wasserstoff, aus denen z.B eine Fischer-Tropsch Herstellung ausgeführt werden kann, bei der entsprechende höhermolekulare Verbindungen wie Alkene, Alkohol usw. gebildet werden. Außerdem könnten die Edukte auch Kohlenwasserstoffe wie Alkene sein, die mit der aufbauenden Reaktion in noch höhere höhermolekulare Verbindungen wie Aldehyd umgesetzt werden können. Der Produkte kann Oxygenate wie DME, Ethanol, Aceton sein und kann auch Kohlenwasserstoff wie Ethen, Propen sein.

Diese aufbauende Reaktion handelt sich um z.B. die Methanolherstellung aus Synthesegas (CO+2H₂->CH₄O), die Fischer-Tropsch-Synthese, die Hydroformylierung von Alkenen zur Erzeugung von Aldehyden/Ketonen (Alken + H₂+CO -> Aldehyd, H₂:CO = 1, kein CO₂) sowie die Hydroformylierung von Alkenen zur Erzeugung von Alkoholen (Alken + 2H₂+CO -> Alkohol, H₂:CO = 2, kein CO₂)

Die Rückführung von CO₂ in die Synthesegas-Erzeugung ist insbesondere vorteilhaft, wenn ein CO-reiches Synthesegas erwünscht ist. Solche Synthesegas-Technologien betreffen insbesondere die Dampfreformierung mit CO₂-Import sowie die Trockenreformierung.

Die oben beschriebene Erfindung wird nachfolgend beispielhaft anhand der DME Herstellung unter Bezugnahme auf die zugehörigen Zeichnungen, die bevorzugte Ausgestaltungen zeigen, detailliert erläutert. Die Erfindung wird durch die rein schematischen Zeichnungen in keiner Weise beschränkt. Es wird dargestellt in
- Fig. 1:: ein Verfahren, bei dem die RTSA stromab der Trenneinrichtung vorgesehen ist;
- Fig. 2:: eine Ausführungsform des erfindungsgemäßen Verfahrens, bei dem die RTSA stromab der Trenneinrichtung und der Synthesegasaufbereitung sowie stromauf des DME Reaktores vorgesehen ist;
- Fig. 3:: ein Verfahren, bei dem die RTSA stromab der DME-Trenneinrichtung vorgesehen ist; und
- Fig. 4:: ein Verfahren, bei dem die RTSA stromab der DME-Trenneinrichtung vorgesehen ist.

Die vorliegende Erfindung betrifft unterschiedliche Anordnungen der oben beschriebenen RTSA in einer DME-Direktsynthese in einem DME-Reaktor 104 basierend auf einem H₂- und CO-haltigen Synthesegas 2, das in einem Synthesegas-Reaktor 101 erzeugt wird (z.B. durch Dampfreformierung eines CH₄-haltigen Einsatzes 1) und sodann konditioniert, d.h. gekühlt und/oder verdichtet wird.Hierbei ist anzumerken, dass die Verdichtung des Synthesegases in dieser Anordnung auch hinter der RTSA erfolgen kann. Beide Anordnungen haben Vorteile: Wird zuerst verdichtet und dann die RTSA durchgeführt, hat das Synthesegas einen höheren Druck als der Reformer. CO₂ kann ohne weiteren Kompressor zum Reformer rezykliert werden; die Adsorber können kleiner ausfallen. Wird zuerst adsorbiert und sodann verdichtet, wird weniger Energie verbraucht. Durch die auftretenden Druckverluste muss dann das CO₂ jedoch mit einem separaten Kompressor auf Reformerdruck verdichtet werden. In beiden Varianten erfolgt bevorzugt die Kühlung und Wasserabscheidung vor der RTSA.

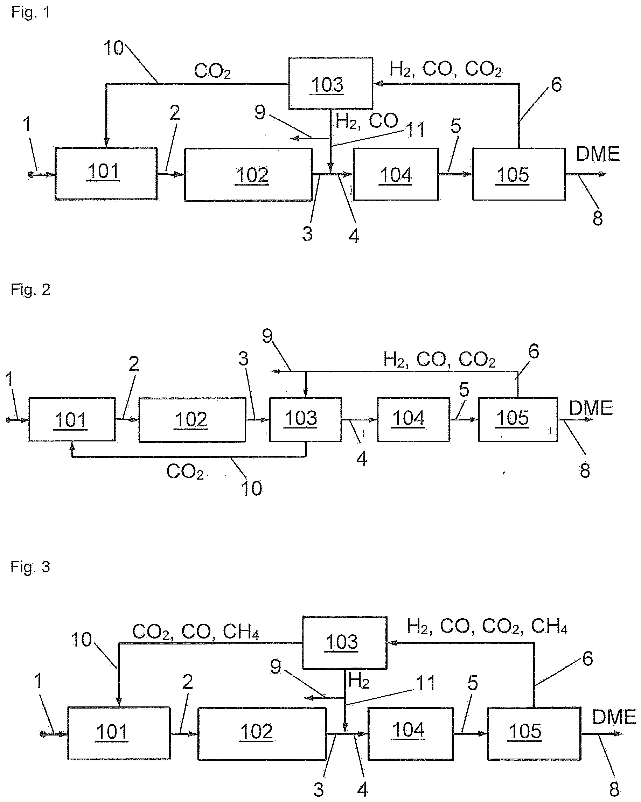

In der Figur 1 wird das von der Synthesegaskühlung- und Aufbereitung 102 aufbereitete Synthesegas 3 einem DME-Reaktor 104 zugeführt. Stromab des DME-Reaktors 104 wird der Produktstrom 5 der DME-Synthese 104 in einer Trenneinrichtung 105 in einen ersten DME-reichen Strom (DME-Produkt) 8 sowie einen zweiten Strom 6, der CO2 sowie nicht umgesetztes Synthesegas (H2 und CO) enthält, getrennt.

Der erste Strom 8 kann auch MeOH sowie H2O enthalten, wobei MeOH und H2O nachfolgend vom ersten Strom 8 abgetrennt werden können. Hierbei kann MeOH an geeigneter Stelle im Prozess rezykliert werden.

Der zweite Strom 6 wird zu der RTSA-Einrichtung 103 geführt. In der RTSA-Einrichtung 103 wird CO2 aus dem zweiten Strom 6 abgetrennt und dann in die Synthesegasreaktor (z.B. Dampfreformierung von CH4) 101 zurückgeführt. Der von CO2 gereinigte Strom 11 aufweisend H2 und CO wird teilweise in den DME-Reaktor 104 zurückgeführt oder verworfen 9 (z.B. um die Anreicherung von leichten Inerten wie N₂ im Recycle zu reduzieren).

Der CO2-reiche Strom 10 wird dabei mittels der RTSA-Einrichtung 103 auf hohem Druck zurückgewonnen. Hierdurch ist es möglich, den CO₂-Gehalt des DME-Synthese-Einsatzes 4 wie gewünscht einzustellen, wobei auf eine Rekompression des rückzuführenden CO₂ verzichtet werden kann oder der Rekompressionsaufwand im Vergleich zum Stand der Technik (Amin- oder Rectisolwäsche) signifikant reduziert werden kann.

In der Figur 2 ist eine erfindungsgemäße Anordnung der RTSA-Einrichtung 103 vorzusehen, bei der der aus der RTSA-Einrichtung 103 entnommene Strom, von dem CO2 abgetrennt wurde, im Kreislauf dem DME-Reaktor 104 zugeführt wird und das aufbereitete Synthesegas 3 vor der Einleitung in den DME-Reaktor 104 in die RTSA-Einrichtung 103 geleitet wird. Die RTSA-Einrichtung 103 wird stromauf des DME-Reaktors 104 sowie stromab der Synthesegaskühlung- und Aufbereitung 102 und gleichzeitig auch stromab der Trenneinrichtung 105 angeordnet. Der aus der Trenneinrichtung 105 abgetrennte zweite Strom 6 wird der RTSA-Einrichtung 103 zugeführt oder verworfen 9. In der RTSA-Einrichtung 103 wird CO2 aus dem zweiten Strom 6 abgetrennt und dann in die Synthesegasreaktor 101 zurückgeführt. Eine solche Anordnung der RTSA-Einrichtung 103 bietet sich z.B an, wenn das Verhältnis H₂:CO des in den DME-Reaktor 104 einzuspeisenden Synthesegases im Bereich von 1 bis 2 liegen soll und vergleichsweise wenig CO₂ zum DME Reaktor 104 gefahren werden soll.

In der Figur 3 wird die RTSA-Einrichtung 103 gleich wie bei der Figur 1 angeordnet. Die Unterschied ist, dass alle kohlenstoffhaltigen Komponenten in dem zweiten Strom 6, wie z.B. CO₂, CO und CH₄ in der RTSA 103 abgetrennt werden und dann als kohlenstoffreiche Ströme 10 in die Synthesegaserzeugung 101 zurückgeführt werden. Der von den kohlenstoffreichen Strömen 10 abgetrennte Strom 11 aufweisen H2 wird teilweise in den DME-Reaktor 104 zurückgeführt oder verworfen 9.

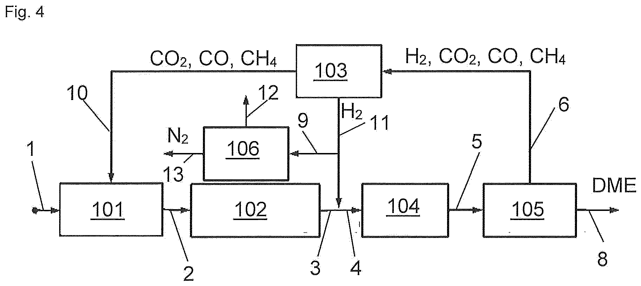

Gemäß Figur 4 kann der H₂-reiche zweite (Purge-) Strom 9 verbrannt werden oder (ganz oder teilweise) weiter aufgereinigt werden, z.B. unter Verwendung einer Druckwechseladsorption 106, um technisch reinen Wasserstoff 12 als Nebenprodukt der DME-Synthese 104 zu gewinnen (z.B. bei einem Druck von 30 bis 60 bar). Der zweite Strom 9 kann bei Bedarf immer aufgereinigt werden. Weiterhin besteht immer die Möglichkeit, einen Teil des Stromes zur RTSA 103 an der RTSA 103 im Bypass vorbeizuführen, um die Zusammensetzung des Synthesegases zu kontrollieren. Dabei darf im Synthesegas insbesondere ein Teil des CO₂ verbleiben, insbesondere im Bereich von 100ppm bis 5 Vol-%.

Die in der Figuren beschriebenen Anordnungen sind nicht nur für DME-Synthese geeignet sondern auch für andere aufbauende Reaktion, bei der aus den niedermolekularen Verbindungen eines Synthesegases zumindest teilweise höhermolekulare Verbindungen gebildet werden.

**Bezugszeichenliste**

| | |
|---|---|
| 1 | Einsatz für Synthesegaserzeugung aufweisend z.B. CH₄ |
| 2 | Syntheserohgas |
| 3 | Aufbereitetes Synthesegas |
| 4 | Synthesegas |
| 5 | Produktstrom oder erster Strom |
| 6 | Zweiter Strom |
| 8 | DME-Produkt |
| 9 | Purge |
| 10 | Zurückgeführter Strom |
| 11 | zweiter Strom |
| 12 | Wasserstoffprodukt |
| 101 | Synthesegas-Reaktor |
| 102 | Synthesegas-Kühlung und Aufbereitung |
| 103 | RTSA-Einrichtung |
| 104 | DME-Reaktor |
| 105 | Trenneinrichtung |
| 106 | Druckwechseladsorption |

## Patentansprüche

1. Verfahren zur Herstellung eines oder mehrerer Reaktionsprodukte mittels einer aufbauenden Reaktion, bei der aus niedermolekularen Verbindungen eines Synthesegases (3) zumindest teilweise höhermolekulare Verbindungen gebildet werden, wobei die niedermolekulare Verbindung eine Klasse von Stoffen mit niedriger Molekülmasse ist, die niedriger Molekülmasse als die höhermolekulare Verbindung haben, aufweisend die Schritte:
- Bereitstellen des Synthesegases (3) aufweisend CO und H₂
- Einleiten zumindest eines Teils des Synthesegases (3) in einen Reaktor (104) sowie Durchführen der aufbauenden Reaktion in dem Reaktor (104), wobei ein Produktstrom (5) enthaltend die höhermolekularen Verbindungen, CO₂, CO und H₂ erzeugt wird,
- Trennen des Produktstromes (5) in einer Trenneinrichtung (105) in einen ersten Strom (8) aufweisend die höhermolekularen Verbindungen sowie in einen zweiten Strom (6) aufweisend CO₂, CO, H₂
wobei zumindest CO₂ in einer Temperaturwechseladsorptionseinrichtung (103) stromab der Trenneinrichtung(105) mittels Temperaturwechseladsorption aus dem zweiten Strom (6) abgetrennt wird, wobei bei der Temperaturwechseladsorption das CO₂ an zumindest einem Adsorbens adsorbiert wird und das mindestens eine mit CO₂ beladene Adsorbens durch Heizen des mindestens einen Adsorbens regeneriert wird, wobei CO₂ desorbiert wird, und wobei das mindestens eine beladene Adsorbens geheizt wird, indem zumindest Wärme eines fluiden Wärmeträgermediums indirekt auf das Adsorbens übertragen wird. wobei der aus der Temperaturwechseladsorptionseinrichtung (103) entnommene Strom, aus dem CO2 abgetrennt wurde, im Kreislauf dem Reaktor (104) zugeführt wird, **dadurch gekennzeichnet, dass** das Synthesegas (3) vor der Einleitung in den Reaktor (104) in die Temperaturwechseladsorptionseinrichtung (103) geleitet wird.

2. Verfahren nach einem der vorhergehenden Ansprüchen, **dadurch gekennzeichnet, dass** das Synthesegas in einem Synthesegas-Reaktor gewonnen wird und dass das bei der Temperaturwechseladsorption (103) adsorbierte CO₂ (10) in den Synthesegas-Reaktor (101) zurückgeführt wird und/oder für andere Anwendungen gewonnen wird.

3. Verfahren nach einem der vorhergehenden Ansprüchen, **dadurch gekennzeichnet, dass** das CO₂ bei der Temperaturwechseladsorption (103) auf einem hohen Druck im Bereich von 5 bar bis 40 bar, bevorzugt 15 bar bis 25 bar, zurückgewonnen wird.

4. Verfahren nach einem der vorhergehenden Ansprüchen **dadurch gekennzeichnet, dass** der von CO₂ gereinigte zweite Strom (11) aufweisend CO und H₂ zumindest teilweise in den Reaktor (104) zurückgeführt wird.

5. Verfahren nach einem der vorhergehenden Ansprüchen, **dadurch gekennzeichnet, dass** in der Temperaturwechseladsorptionseinrichtung (103) neben CO₂, auch CO und CH₄ mittels Temperaturwechseladsorption aus dem zweiten Strom (6) abgetrennt werden und in den Synthesegas-Reaktor (101) zurückgeführt werden, wobei der von CO₂, CO und CH₄ gereinigte zweite Strom (11) zumindest teilweise in den Reaktor (104) zurückgeführt wird und/oder zumindest teilweise in eine Druckwechseladsorptionseinrichtung (106) geleitet wird und dort mittels Druckwechseladsorption weiter gereinigt wird (12), wobei N₂ sowie auch kohlenstoffhaltigen Komponenten adsorbiert werden.

6. Verfahren nach einem der vorhergehenden Ansprüchen, **dadurch gekennzeichnet, dass** die niedermolekularen Verbindungen Kohlenmonoxid und Wasserstoff umfassen.

7. Verfahren nach einem der vorhergehenden Ansprüchen, **dadurch gekennzeichnet, dass** die höhermolekularen Verbindungen Oxygenate sind.

8. Verfahren nach einem der vorhergehenden Ansprüchen, **dadurch gekennzeichnet, dass** die höhermolekulare Verbindungen Kohlenwasserstoffe sind.

9. Verfahren nach einem der vorhergehenden Ansprüchen, **dadurch gekennzeichnet, dass** die aufbauende Reaktion eine DME-Herstellung ist.

10. Verfahren nach einem der vorhergehenden Ansprüchen, **dadurch gekennzeichnet, dass** die aufbauende Reaktion eine Fischer-Tropsch-Herstellung ist.

11. Verfahren nach einem der vorhergehenden Ansprüchen, **dadurch gekennzeichnet, dass** die aufbauende Reaktion eine Methanol-Herstellung ist.

12. Verfahren nach einem der vorhergehenden Ansprüchen, **dadurch gekennzeichnet, dass** die aufbauende Reaktion eine Hydroformylierung von Alkenen zu Aldehyden oder Ketonen aufweist.

## Claims

1. Process for producing one or more reaction products by means of a build-up reaction, in which higher-molecular-weight compounds are formed at least in part from low-molecular-weight compounds of a synthesis gas (3), wherein the low-molecular-weight compound is a class of substances having a low molecular mass which have lower molecular masses than the higher-molecular-weight compound, comprising the steps:
- providing the synthesis gas (3) comprising CO and H₂
- introducing at least a part of the synthesis gas (3) into a reactor (104) and also carrying out the build-up reaction in the reactor (104), wherein a product stream (5) containing the higher-molecular-weight compounds, CO₂, CO and H₂ is generated,
- separating the product stream (5) in a separation appliance (105) into a first stream (8) having the higher-molecular-weight compounds, and also into a second stream (6) comprising CO₂, CO, H₂
wherein at least CO₂ is separated off from the second stream (6) in a temperature-swing adsorption appliance (103) downstream of the separation appliance (105) by means of temperature-swing adsorption, wherein, in the temperature-swing adsorption, the CO₂ is adsorbed to at least one adsorbent and the at least one CO₂-loaded adsorbent is regenerated by heating the at least one adsorbent, wherein CO₂ is desorbed, and wherein the at least one loaded adsorbent is heated by transferring at least heat of a fluid heat-carrier medium indirectly to the adsorbent, wherein the stream that is withdrawn from the temperature-swing adsorption appliance (103) and from which the CO₂ was separated off is fed in the circuit to the reactor (104), **characterized in that** the synthesis gas (3), before it is introduced into the reactor (104), is passed into the temperature-swing adsorption appliance (103).

2. Process according to any one of the preceding claims, **characterized in that** the synthesis gas is obtained in a synthesis gas reactor and **in that** the CO₂ (10) that is adsorbed in the temperature-swing adsorption (103) is recirculated to the synthesis gas reactor (101) and/or is obtained for other uses.

3. Process according to any one of the preceding claims, **characterized in that** the CO₂ is recovered in the temperature-swing adsorption (103) at a high pressure in the range from 5 bar to 40 bar, preferably 15 bar to 25 bar.

4. Process according to any one of the preceding claims, **characterized in that** the second stream (11) purified by removing CO₂ and comprising CO and H₂ is at least in part recirculated to the reactor (104).

5. Process according to any one of the preceding claims, **characterized in that**, in the temperature-swing adsorption appliance (103), in addition to CO₂, CO and CH₄ are also separated off from the second stream (6) by means of temperature-swing adsorption and recirculated to the synthesis gas reactor (101), wherein the second stream (11) that is purified by removing CO₂, CO and CH₄ is at least in part recirculated to the reactor (104) and/or is passed at least in part to a pressure-swing adsorption appliance (106) and there further purified (12) by means of pressure-swing adsorption, wherein N₂ and also carbonaceous components are adsorbed.

6. Process according to any one of the preceding claims, **characterized in that** the low-molecular-weight compounds comprise carbon monoxide and hydrogen.

7. Process according to any one of the preceding claims, **characterized in that** the higher-molecular-weight compounds are oxygenates.

8. Process according to any one of the preceding claims, **characterized in that** the higher-molecular-weight compounds are hydrocarbons.

9. Process according to any one of the preceding claims, **characterized in that** the build-up reaction is a DME preparation.

10. Process according to any one of the preceding claims, **characterized in that** the build-up reaction is a Fischer-Tropsch preparation.

11. Process according to any one of the preceding claims, **characterized in that** the build-up reaction is a methanol preparation.

12. Process according to any one of the preceding claims, **characterized in that** the build-up reaction comprises a hydroformylation of alkenes to form aldehydes or ketones.

## Revendications

1. Procédé de fabrication d'un ou de plusieurs produits de réaction au moyen d'une réaction d'édification, selon lequel des composés de poids moléculaire élevé sont formés au moins en partie à partir de composés de faible poids moléculaire d'un gaz de synthèse (3), le composé de faible poids moléculaire correspondant à une classe de substances de faible masse moléculaire qui ont une masse moléculaire plus faible que le composé de poids moléculaire élevé, comprenant les étapes suivantes :
- la préparation du gaz de synthèse (3) comprenant du CO et de l'H₂,
- l'introduction d'au moins une partie du gaz de synthèse (3) dans un réacteur (104) et la réalisation de la réaction d'édification dans le réacteur (104), un courant de produits (5) qui contient des composés de poids moléculaire élevé, du CO₂, du CO et de l'H₂ étant formé,
- la séparation du courant de produits (5) dans un dispositif de séparation (105) en un premier courant (8) comprenant les composés de poids moléculaire élevé et en un deuxième courant (6) comprenant du CO₂, du CO, de l'H₂, au moins le CO₂ étant séparé du deuxième courant (6) par adsorption modulée en température dans un dispositif d'adsorption modulée en température (103) disposé en aval du dispositif de séparation (105) ; lors de l'adsorption modulée en température, le CO₂ étant adsorbé sur au moins un adsorbant et ledit au moins un adsorbant chargé en CO₂ étant régénéré par chauffage dudit au moins un adsorbant, le CO₂ étant désorbé, et ledit au moins un adsorbant chargé étant chauffé au moins par transfert indirect de la chaleur d'un milieu caloporteur fluide à l'adsorbant, le courant soutiré du dispositif d'adsorption modulée en température (103), à partir duquel du CO₂ a été séparé, étant introduit dans un circuit dans le réacteur (104), **caractérisé en ce que** le gaz de synthèse (3) est introduit dans le dispositif d'adsorption modulée en température (103) avant l'introduction dans le réacteur (104).

2. Procédé selon l'une quelconque des revendications précédentes, **caractérisé en ce que** le gaz de synthèse est obtenu dans un réacteur de gaz de synthèse, et **en ce que** le CO₂ (10) adsorbé lors de l'adsorption modulée en température (103) est recyclé dans le réacteur de gaz de synthèse (101) et/ou récupéré pour d'autres applications.

3. Procédé selon l'une quelconque des revendications précédentes, **caractérisé en ce que** le CO₂ est récupéré lors de l'adsorption modulée en température (103) à une pression élevée dans la plage allant de 5 bar à 40 bar, de préférence de 15 bar à 25 bar.

4. Procédé selon l'une quelconque des revendications précédentes, **caractérisé en ce que** le deuxième courant (11) débarrassé du CO₂, comprenant du CO et de l'H₂, est au moins partiellement recyclé dans le réacteur (104).

5. Procédé selon l'une quelconque des revendications précédentes, **caractérisé en ce qu'**en plus du CO₂, du CO et du CH₄ sont également séparés du deuxième courant (6) dans le dispositif d'adsorption modulée en température (103) par adsorption modulée en température, et recyclés dans le réacteur de gaz de synthèse (101), le deuxième courant (11) débarrassé du CO₂, du CO et du CH₄ étant au moins partiellement recyclé dans le réacteur (104) et/ou au moins partiellement introduit dans un dispositif d'adsorption modulée en pression (106), et y étant davantage purifié (12) par adsorption modulée en pression, du N₂ ainsi que des composants carbonés étant adsorbés.

6. Procédé selon l'une quelconque des revendications précédentes, **caractérisé en ce que** les composés de faible poids moléculaire comprennent du monoxyde de carbone et de l'hydrogène.

7. Procédé selon l'une quelconque des revendications précédentes, **caractérisé en ce que** les composés de poids moléculaire élevé sont des composés oxygénés.

8. Procédé selon l'une quelconque des revendications précédentes, **caractérisé en ce que** les composés de poids moléculaire élevé sont des hydrocarbures.

9. Procédé selon l'une quelconque des revendications précédentes, **caractérisé en ce que** la réaction d'édification est une fabrication de DME.

10. Procédé selon l'une quelconque des revendications précédentes, **caractérisé en ce que** la réaction d'édification est une fabrication de Fischer-Tropsch.

11. Procédé selon l'une quelconque des revendications précédentes, **caractérisé en ce que** la réaction d'édification est une fabrication de méthanol.

12. Procédé selon l'une quelconque des revendications précédentes, **caractérisé en ce que** la réaction d'édification est une hydroformylation d'alcènes en aldéhydes ou cétones.
